# EUROPEAN PATENT APPLICATION

(11) **EP 4 732 764 A1**
(43) Date of publication of application: **29.04.2026**
(21) Application number: 25194133.2
(22) Date of filing: 05.08.2025
(51) Int. Cl.: A61B 5/022, A61B 5/00

(54) **BLOOD PRESSURE MONITORING SYSTEM AND METHOD**

(30) Priority: 06.08.2024 NL 2038400
(71) Applicant: Finapres Medical Systems B.V., 7521 PH Enschede (NL)
(72) Inventor: de Jong, Menno, 7521 PH ENSCHEDE (NL); Vos, Johan Siert, 7521 PH ENSCHEDE (NL)
(74) Representative: Arnold & Siedsma

(57) **Abstract**

The invention relates to a blood pressure monitoring system and method, comprising:
- a pressure measurement unit configured for being arranged on a body part of a user and for performing blood pressure measurements at the body part, the pressure measurement unit including at least a controllable element and one or more sensors configured to perform measurements to form data;
- a controller operatively connected to the controllable element for controlling the controllable element based on a configuration of the controller, the configuration including at least one parameter;
- a memory configured for storing a predetermined configuration for the controller,

wherein the controller is configured to:
- control the controllable element based the predetermined configuration stored in the memory,
the blood pressure monitoring system comprising:
- a processor operatively connected to at least the controller and the memory;

wherein the processor is configured to:
a) receive the data from the pressure measurement unit;
b) based on the received data, determine another configuration for the controller, by determining a value for the at least one parameter thereof; and
c) cause the controller to control the controllable element based on the other configuration instead of on the predetermined configuration.

## Description

The invention relates to a blood pressure monitoring system, comprising a pressure measurement unit configured for being arranged on a body part of a user and for performing blood pressure measurements at the body part, the pressure measurement unit includes at least a controllable element and one or more sensors configured to perform measurements to form data, a controller is operatively connected to the controllable element for controlling the controllable element based on a configuration of the controller, the configuration including at least one parameter. The system also includes a memory configured for storing a predetermined configuration for the controller. The controller is configured to control the controllable element based the predetermined configuration stored in the memory.

Blood pressure monitoring systems of this type are generally known, and may be used to monitor a blood pressure while a user goes about his or her daily routine. The blood pressure may thus not be monitored directly, but instead may be stored for later review. Nevertheless, the actual measurement may therefore take place outside a clinical setting, even without the presence of medical or otherwise qualified personnel.

As a result, issues may arise from the pressure measurement unit not being properly arranged on the body part. Such issues may for instance be unreliable measurements and conclusion drawn from such measurements.

It is therefore an object to at least partially address this problem.

According to the invention, the object is achieved by a blood pressure monitoring system as described above, wherein the blood pressure monitoring system comprises:
- a processor operatively connected to at least the controller and the memory;
wherein the processor is configured to:
a) receive the data from the pressure measurement unit;
b) based on the received data, determine another configuration for the controller, by determining a value for the at least one parameter thereof; and
c) cause the controller to control the controllable element based on the other configuration instead of on the predetermined configuration.

Accordingly, the processor brings about a correction of the control behaviour, via the other configuration, which may result in better blood pressure measurements. By basing the correction on the measurements taken by the measurement unit (i.e. the data), the correction can be made based on the current circumstances, such as the orientation in which the blood pressure monitoring system is applied, or based on user-specific circumstances. Both of these examples would otherwise be impossible to account for.

The controllable element may be an actuatable element, that e.g. moves, or changes shape or volume when controlled by the controller.

The controller may be a hardware or a software module. The controller may be implemented as part of, or as running on, the processor. It is thus not necessary that the processor and the controller are different entities - either physical or embodied in software, firmware or otherwise.

The other configuration may be a configuration of the same type as the predefined configuration, i.e. being usable for the same function, however being different in the sense that the value for the at least one parameter is different. In particular, the other configuration is equal in type and different in the value of the at least one parameter. It is noted that the at least one parameter is not necessarily a numerical parameter. As an example, the at least one parameter may include an indication of a type of control strategy to be employed by the controller. As a rudimental example the at least one parameter may be used to signal whether the controller is to apply PID or PI based control (notwithstanding that strictly speaking the latter could also be achieved by setting the parameters of a PID controller).

The particular preferred control method for applied by the controller is indirect adaptive control. Preferably, the controller is thus arranged to determine and adjust control parameters, such as said at least one parameter based on parameter estimation of operating parameters, which may also be control parameters, of the controllable element.

In general, a blood pressure measurement unit with a controllable element is known. In such a case the controller may be provided to control the controllable element. The controller is tuned beforehand to the blood pressure measurement unit and the intended use as best as possible, so that the controllable element performs the desired behaviour as best as possible. The configuration for the controller (predefined or the other configuration) may thus include the controlling tuning, or even equal the controller tuning. According to the invention, the controller may be updated, i.e. adjusted or retuned, based on the other configuration.

When the controller is well-tuned, for instance when a more optimal configuration is applied, the quality of the measurements taken using the blood pressure measurement unit may be higher and may therefore be of higher diagnostic relevance.

In that sense, step b) may comprise system identification, in particular online (i.e. while the controller is being used). Step b) may thus comprise determining the other configuration as the configuration corresponding to the controller being tuned to the identified system. The system to be identified may include the controllable element and its relevant surroundings, such as the body part upon which it acts.

An exemplary type of blood pressure monitoring system, to which the current disclosure can be applied is a volume-clamp based blood pressure monitoring system, for instanced based on research by Peńa̋zJ. Peńa̋z, Photoelectric measurement of blood pressure, volume and flow in the finger, IMBE 1973 Dresden, 7-2 page 104. In such volume-clamp applications, a light source and receptor are used to measure blood flow in the finger. A light signal modified by the finger is received at the receptor, and the modification of it is indicative of blood flow in the finger. Put more exactly, the light signal is modified by the arteries in the finger, which change shape according to their internal pressure, the elasticity of the artery wall, and the external pressure. At the same time, a gas bladder is used to push on the finger. Using the controller, the bladder is filled so as to compensate for blood flow in the finger, so that ideally, the light signal remains unchanged. In this ideal case, the pressure in the bladder is representative of the blood pressure. A blood pressure measurement unit as described herein may thus be of the known volume-clamp type. It is noted that ideally, the light signal is kept unchanged at a specific setpoint, which may be set according to the Physiocal algorithm, also described by Peńa̋z and later expanded upon by others.

Preferably, in use, the volume of the gas bladder is estimated by the controller through parameter estimation, for example as part of the aforementioned adaptive control method. In combination with previously obtained test data, the estimated bladder volume may be used to determine whether the bladder volume is too high or too low compared to its desired setpoint.

It can be seen that if the controller for controlling the bladder volume is optimized in the sense that it achieves more accurately its desired setpoint, the pressure reading in the bladder corresponds more closely to the actual blood pressure, so that the pressure measurements are more accurate.

It should however be noted that the current disclosure is applicable also to other types of blood pressure monitoring systems that have a controllable element and a controller therefor.

The processor may be configured to determine a blood pressure of the user based on the data. Accordingly, a more accurate blood pressure reading may be obtained based on the data, which may be more accurate due to the updated or optimized controller. In the case of a volume-clamp system, the blood pressure reading may be obtained by measuring the gas pressure in the bladder while controlling the bladder to keep the light signal at the calibrated setpoint.

The applicant has realized that the ideal configuration for the controller has a relation to the physical system including at least the controllable element, and for instance the body part upon which the controllable element acts. In other words, the ideal configuration has a relation to the conditions under which measurements are taken. Surprisingly, the applicant has found that therefore, a relatively large deviation between a reference configuration and a newly determined more optimal configuration may be indicative of possibly sub-optimal conditions. These conditions may negatively influence the measurements being taken, which therefore become less reliable and/or accurate.

To make use of this principle, the processor may be configured to e) determine an indicator of the quality of the data based on the other configuration.

The indicator may be used in a variety of ways. As an example, the indicator may be stored or output together with the blood pressure measurements, so that a physician or other person interpreting the measurement data can put unexpected measurements, such as outliers, etc. into the perspective of reduced quality. The applicant has found that this is highly relevant, as it is often the unexpected measurement results that are used for diagnostic purposes. The processor may thus be configured to control a or the output module to output the blood pressure simultaneous with the corresponding indicator for the quality.

The indicator may be an indicator of the quality of the data, in particular of blood pressure measurements taken using the pressure measurement unit.

The prevailing theory, although not relevant in terms of scope of the invention, is that if the configuration needs adjusting in relatively extreme proportions to account for the conditions of the measurements, these conditions are not likely to facilitate reliable measurements. The applicant has thus realized that the deviation of the configuration from a reference may be taken as an indicator representative of quality of measurements (herein called the indicator of quality). After all, a measurement that is not likely to be reliable, is of low quality. During analysis, the quality of a signal or measurement is of particular importance. An unreliable measurement may for instance include a faulty measurement, i.e. a reading that is relatively far removed from the physical quantity being measured. At the same time deviations from a normal behaviour are often the focus of medical personnel, researchers, etc. As such, it is of significant value to be able to determine whether or not an outlier or otherwise unexpected behaviour is likely to have been caused by unreliable measurements, or is indicative of some condition or is otherwise of interest. In other words, medical personnel may be aided in their work if they are provided with an indicator of the quality of the of the measurements involved.

As another example of the useful application of the quality indicator, the indicator may be used to further increase the quality of the measurements taken, by indicating to the user when a low quality is detected. Accordingly, the user is able to take countermeasures to ensure higher quality measurements. For this reason, the blood pressure monitoring system may comprise an output module. Then, the processor may be operatively connected to the output module and may be configured to:
f) control the output module to output the indicator for quality.

A user may use the indicator to e.g. change the conditions under which the measurements are taken, in order to guarantee higher quality results.

As an example, the indicator of quality may be given if and only if the quality is lacking. A lacking quality may for instance be determined by comparing the indicator as a numerical value to a predefined quality threshold.

The output module may be a visual and/or auditory output module. As an example, the output module may be a display. As another example, that can be combined with the previous, the output module may comprise one or more LEDs. As another example, that can be combined with any of the previous examples, the output module comprises a sound generator, such as a speaker or ringer.

The indicator may be determined by comparing the other configuration (i.e. the configuration which is determined based on measurements taken) with a predefined configuration as a reference, such as the predetermined configuration, a predefined threshold, etc. the outcome of the comparison may be used as the indicator of the quality. In practice, the indicator may be determined by comparing the at least one parameter of the other configuration to a predefined threshold, such as the at least one parameter of the predefined configuration.

In reaction to an indication of low quality, the user may take preventive measures to increase the quality of the measurement. Accordingly, even if no medical or otherwise qualified personnel is around, a user may be able to obtain high quality measurements.

Referring to the exemplary application of the volume-clamp type monitoring system, the following is offered as further elucidation, but not limitation unless explicitly stated in the claims. In the case of the volume-clamp measurement unit, the required volume in the bladder for achieving the calibrated setpoint depends not only on the blood pressure inside the vessels of the user's finger, but to a relatively large extent on the arrangement of the cuff of such a volume-clamp unit on the finger. Depending on e.g. the orientation of the cuff with respect to the finger, the volume may need to be increased or decreased in order to provide a desired pressure. At the same time, the total volume of the cuff is highly relevant for the behavior of the controller. As an example, if the controller is set to change the volume by a relatively small amount in respond to a deviation from the setpoint, whilst the bladder volume is relatively large, the control system as a whole may act relatively slow. The opposite situation is also problematic: if the controller is set to change the volume by a relatively large amount, whilst the volume itself is relatively small, the controlled system is likely to overshoot the setpoint (i.e. it operates too fast). By providing a more optimized configuration for the controller, the conditions in which the measurements are being taken may be compensated for. Accordingly, the measurement quality may be increased. In much the same way, required optimization - e.g. in terms of a deviation from a reference - is a measure for how sub-optimal the conditions are, and thus for the quality of the measurements. When the user is notified of the low quality, the user may be prompted to rearrange the finger cuff, for instance by straightening it with respect to the finger, resulting in a more optimal required bladder volume. This may result in a smaller required deviation from the reference, i.e. the newly determined configuration (the other configuration in terms of the claims) is closer to the predefined configuration, thus indicating a higher quality. The measurements quality overall can thus be improved by notifying to the user of a low quality, who can take preventive actions.

The same principle can be applied to other relevant parameters, and the cuff volume is thus in principle exemplary only. It is even possible to apply the principles set out herein to other types of blood pressure monitoring systems.

It is further noted that it is in principle possible to use the indicator of quality without actually using the new configuration for the controller. A relatively elegant system, may thus be that of claim 1, wherein step c) is not necessary. In contrast, determining the indicator of the quality based on the configuration in itself can also aid to improve quality. The other features described herein can thus be applied with or without the subject matter of step c), be it in terms of the blood pressure monitoring system or the method as described herein.

As explained, the indicator of quality may be useful for later analysis. For this reason, the processor may cause the quality indicator to be stored, for instance for later retrieval, for instance in the memory. This can be done in addition to outputting the indicator or instead of outputting it (directly). In either case, the output module may be a communications device, such as part of the processor or between the processor and the memory, that allows outputting the determined quality indicator to the memory for storage. When needed, the processor - or some external device - may retrieve the quality indicator from the memory. Of course, the memory for storing the quality indicator need not necessarily be the same memory that stores the model and/or measurements.

As an alternative, or additionally, the output module may be a user output module. In that case, the quality indicator would be available to a user directly.

The processor may be configured to repeat steps a), b) on the one hand, and c) and/or d) on the other hand, throughout a continuous blood pressure monitoring procedure. As a result, a repeated update of a more optimal configuration for the controller may be obtained while blood pressure measurements are taken as part of the monitoring procedure. Of course, other related steps performed by the processor may also be repeatedly performed, such as steps e) and f). The same goes for the corresponding steps of the method explained below, including step g).

Repeating these steps allow to continuously account for changing conditions, thereby increasing the quality of measurements over a longer period of time even under changing circumstances.

The steps may be repeated each time as a calibration procedure, for instance at initialization and repeated at (possibly varying) time intervals. The calibration procedure may further include determining a setpoint for the controller, such as the Physiocal algorithm.

The blood pressure monitoring system may be portable and/or the pressure measurement unit may comprise a finger cuff, to facilitate the user going about on their daily activities while the blood pressure is being monitored.

In particular, the blood pressure measurement unit may be of the volume-clamp type. Accordingly, the controllable element may comprise a bladder, specifically a gas bladder. The controller may be configured to control a volume of the bladder. The controllable element may in that case be any element that adjusts the volume of the bladder, such as a system of pumps and/or valves.

The one or more sensors may include at least one first sensor of a first type, and at least one second sensor of a second type, the second type being different than the first type.

A pressure measurement unit of this type may perform particularly accurately, since multiple physical quantities can be accounted for in determining a blood pressure.

The controller may be configured to use a value from the at least one second sensor as an input, and wherein the controllable element is configured to influence, together with the body part, the value read by the at least one first sensor.

As a result, the controller can be used to compensate for changing conditions measured by the second sensor, so that the first sensor can measure a quantity uninfluenced by the changing conditions.

As an example, in the volume-clamp system, a light intensity may be used as input for the controller. Light may be used to measure the cross section of the blood vessel(s) in the user's finger. Depending on the pressure in the vessel(s), they expand and contract. When a counterpressure is applied using the bladder, the cross section of the vessels can be kept constant. Then, the first sensor can measure a pressure in the bladder, which at that time is equal to the pressure in the vessels. Accordingly, by suitable control based on the light intensity, the elasticity of the blood vessels can be compensated for in the pressure measurement.

As such, it is particularly practical when the at least one first sensor is configured to measure a pressure inside the bladder and/or when the at least one second sensor is configured to measure an intensity of optionally visible light interacting with the body part. The interaction of light can be any suitable interaction depending on the position of the second sensor and an optional light source. The interaction can for instance be reflection, absorption or transmission, or a combination thereof.

The data - which is used to determine the other configuration for the controller - may comprise a value representative of a physical quantity associated with the controllable element, such as a gas pressure inside the bladder described above. Thus, the configuration may be adjusted based on at least a type of sensor that is different from the controller input. Of course, the controller input may be used as alternative or in addition. In the example of the volume-clamp measurement unit, the other configuration may be determined based on at least the pressure in the bladder, whereas the controller receives light intensity measurements as input.

In any case, the data may comprise pressure data, such as blood pressure data. Accordingly, the disclosure in essence provides controller optimization based on measurements taken with the use of the same controller.

The invention also relates to a method of facilitating blood pressure measurements using a blood pressure measurement unit, the method comprising:
- controlling a controllable element of the blood pressure measurement unit according to a predefined configuration including at least one parameter; and
wherein the method further comprises:
a) receiving data from the blood pressure measurement unit,
b) determining another configuration for the controller based on the received data, by determining a value for the at least one parameter thereof; and
c) causing the controller to control the controllable element based on the other configuration instead of on the predetermined configuration.

Much like the description of the blood pressure monitoring system, the further optimization of the controller by providing a better configuration may increase the quality of measurements taken.

The method may be performed using a blood pressure monitoring system as described herein.

The method may further comprise d) determining a blood pressure of a user based on the data.

The method may further include e) determining an indicator of the quality of the data based on the other configuration. As explained above, the indicator can be used to increase the diagnostic relevance of blood pressure measurements, or to allow a user to adjust the conditions under which the measurements are taken to increase their quality, or both.

To make use of the indicator, the method may comprise f) outputting the indicator of the quality, for instance together with the data - such as blood pressure measurements.

With our without the measurements, the indicator may also be output to a user, for instance as the measurements are being taken. Displays, LEDs or an audible signal are options considered, but alternatives are within scope of the current disclosure.

It may be particularly advantageous if the method further comprises g) determining a suggested action for a user to improve the quality of the blood pressure measurements, for instance based on the other configuration. Optionally, the suggestion is output to e.g. the user.

Accordingly, a user (or similar, such as medical personnel etc.) may be guided according to the method to improve the measurement takings. As an example, if it can be determined from the configuration that the bladder is too large, a suggestion may be to rearrange and/or straighten the cuff so that the bladder fits better to the user's finger. As a result, a user may be provided with advice on how to improve quality of measurements when it is relevant, or even continuously.

Preferably, the suggestion is determined when the indicator of quality is relatively low, e.g. lower than a predetermined threshold.

The method may be performed repeatedly during continuous blood pressure monitoring. As such, the quality of measurements taken over a longer period of time may be known, and account can be taken of changing conditions that may have affected the quality of the measurements in the meantime.

The invention will be further elucidated with reference to the drawings, in which:
Figure 1A schematically shows a blood pressure monitoring system;
Figure 1B schematically shows a pressure measurement unit;
Figure 2 schematically shows a display; and
Figure 3 is a flow chart of a method of facilitating blood pressure monitoring.

Throughout the figures, like elements are referred to using like reference numerals.

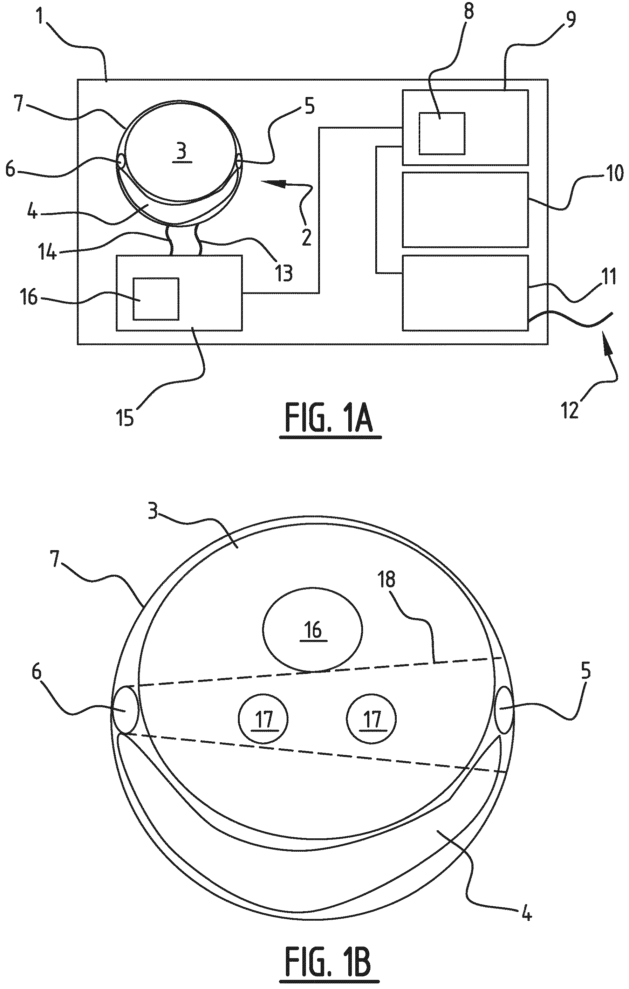

Figure 1A shows a blood pressure monitoring system 1, comprising a pressure measurement unit 2, which has a part 7 that can be arranged on a body part 3. As an example, the part 7 shown is a finger cuff 7 that can be arranged on a finger 3. Details of the cuff 7 are explained below in relation to figure 1B. Included is a controllable element 4, which in the example is a bladder 4 that can be inflated and deflated by a pump 15 connected to it via a pressure lines 13. A communication line 14 is also provided. Of course, other types of controllable elements could have been used. The pump 15 includes a sensor 16.

The pressure monitoring system 1 also includes a controller 8 which, as an example, is run as software module on a processor 9 also part of the blood pressure monitoring system 1. The controller 8 could of course be embodied as a separate piece of hardware. The controller 8 is connected to the blood pressure measurement unit 2 to control the controllable element 4 thereof. the controller 8 functions according to a configuration which includes at least one parameter. The blood pressure monitoring system 1 further includes a memory 10 connected to the processor 9 and an output module 11 connected to the processor 9.

Figure 1B shows a particular type of pressure measurement unit 2, in this case include a finger cuff 7, which will be used as an exemplary type of unit, even though the principles of the current disclosure can be applied to any type of unit with a controllable element 4. The finger cuff 7 includes a bladder 4 which can be expanded and contracted by inserting or releasing air from the pump 15 through pressure lines 13. The pressure measurement unit 2 is portable. The finger cuff 7 also includes a light emitter 6 and a receptor 5. A simplified finger 3 is shown in cross section, including a bone 16 and two arteries 17. The emitter 6 emits light 18 through the finger. The light 18 is modified by interaction with the arteries 17, and then received by the receptor 5. The light signal 5 at the receptor is influenced by the state of the arteries 17. As the blood pressure in the arteries 17 changes, the size of the arteries normally changes. The change in size can be registered by measuring the variation in light 18 with the receptor 5. Using a known calibration algorithm, an unloaded state of the walls of the arteries 17 is determined as a set point for the controller 8. The controller 8 then controls the bladder 4 by instructing the pump 14 to apply a certain pressure. While the size of the arteries 17 is kept constant by inflating and deflating the bladder 4, the gas pressure in the bladder 4 equals the blood pressure inside the arteries 17. The required pressure (either taken at the controller output or measured) is then taken as a blood pressure measurement inside the arteries 17. It can therefore be concluded that the controller 8 takes as an input the reading form the receptor 5 (which is a kind of sensor) and controls the volume in the bladder 4. By controlling the volume, the pressure changes. The pressure reading thus follows from another type of sensor (not shown).

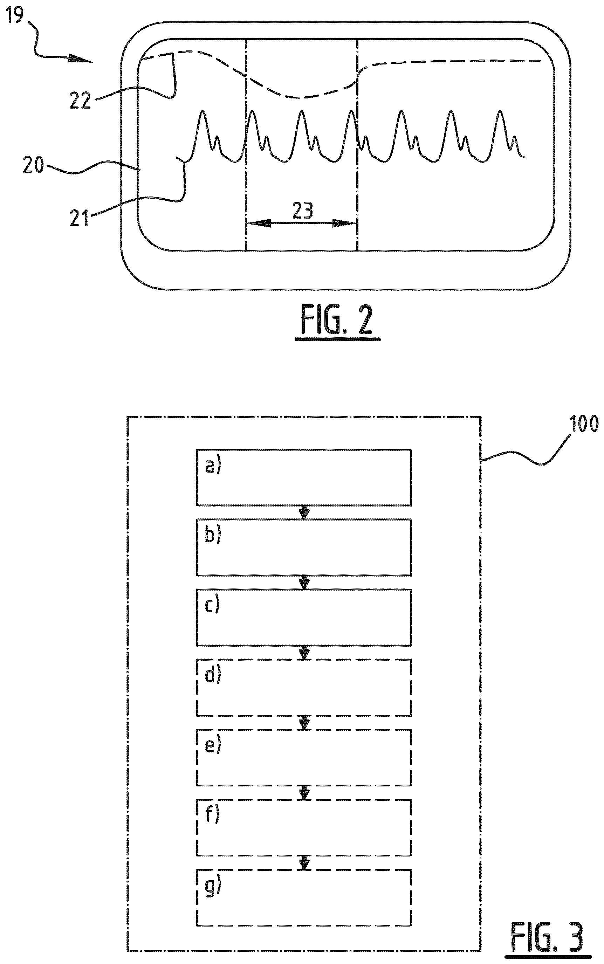

The processor 9 is configured to a) receive data from the pressure measurement unit. Reference is at this point also made to figure 3, which shows a method 100 of facilitating blood pressure monitoring including steps a) - g) which may or may not be performed by the blood pressure monitoring system of figure 1. Steps in dashed lines are optional, and step c) is also optional.

Going back to the processor, it is further configured to perform a step b) of determining another configuration for the controller, based on the received data. In effect, the processor determines a value for the at least one parameter of a new configuration which could for instance in step c) be used to take the place of the configuration according to which the controller 8 currently runs. Accordingly, the controller 8 may be updated, or tuned, as instructed by the processor 9. The processor 9 is thus able to perform system identification and controller tuning, so that the controllable element is controlled more optimally.

The processor 9 is also configured to d) determine a blood pressure of the user based on the data, and to e) determine an indicator of the quality of the data based on the other configuration (i.e. the newly determined configuration, which is based on the data). The indicator may be determined by comparing the newly determined configuration to the predefined configuration, for instance by comparing the respective at least one parameters thereof to each other. A large difference could indicate a low quality and vice versa.

Referring again to figure 1A, the blood pressure monitoring system also includes the output module 11. Any suitable kind of output may be used, but two examples are given herein. As a first example, the output module 11 may be a user output module 11, which outputs to the user the indicator of the quality. For that purpose the user output module 11 may included e.g. LEDs, a speaker or other audio device, a display, or a combination of those. As a second example, the output module 11 may be communications module 11, which can be connected to an external appliance via a connection 12 (either wired or wireless), to output the indicator for display and/or analysis and/or storage.

The processor 9 may be configured to f) control the output module 11 to indicator of quality, preferably together with blood pressure measurement results.

Ideally, the processor 9 repeats these steps, while continuously monitoring a blood pressure of a user.

As an exemplary way of displaying the indicator for the quality, reference is made to figure 2 which shows a display 19 with a screen 20. The screen 19 shows to for example a user in realtime, or to a e.g. medical personal afterwards for evaluation, the measured blood pressure 21 as a continuous signal. The indicator for reliability can for instance be shown as a further data line 22 the level of which corresponds to the quality. Alternatively or additionally, measurements of relatively low quality can be highlighted using e.g. a coloured section 23 of the screen. Of course, other methods may be used to display the indicator 22 together with blood pressure data 21, preferably simultaneously.

Finally, reference is made to figure 3, showing in a flow chart a method of facilitating blood pressure measurements using a blood pressure measurement unit. The method 100 comprises controlling a controllable element of the blood pressure measurement unit according to a configuration including at least one parameter, in this case preferably continuously or repeatedly. Then, the method comprises steps a) - g). All but step g) were explained above, and may according to the current disclosure be performed by any suitable device. Step g) includes determining a suggested action for a user to improve the quality of the blood pressure measurements, and optionally outputting the suggestion to e.g. the user.

While the invention has been explained above with reference to particular embodiments and examples, the invention is not limited thereto. The following claims instead define the scope of the invention.

## Claims

**1.** Blood pressure monitoring system, comprising:
- a pressure measurement unit configured for being arranged on a body part of a user and for performing blood pressure measurements at the body part, the pressure measurement unit including at least a controllable element and one or more sensors configured to perform measurements to form data;
- a controller operatively connected to the controllable element for controlling the controllable element based on a configuration of the controller, the configuration including at least one parameter;
- a memory configured for storing a predetermined configuration for the controller,
wherein the controller is configured to:
- control the controllable element based the predetermined configuration stored in the memory,
the blood pressure monitoring system comprising:
- a processor operatively connected to at least the controller and the memory;
wherein the processor is configured to:
a) receive the data from the pressure measurement unit;
b) based on the received data, determine another configuration for the controller, by determining a value for the at least one parameter thereof; and
c) cause the controller to control the controllable element based on the other configuration instead of on the predetermined configuration.

**2.** Blood pressure monitoring system according to the previous claim, wherein the processor is further configured to:
d) determine a blood pressure of the user based on the data.

**3.** Blood pressure monitoring system according to any of the preceding claims, wherein the processor is further configured to:
e) determine an indicator of the quality of the data based on the other configuration.

**4.** Blood pressure monitoring system according to the previous claim, wherein step e) comprises comparing the other configuration to a predefined configuration, such as the predetermined configuration, and using the outcome of the comparison as the indicator of the quality.

**5.** Blood pressure monitoring system according to the previous claim, wherein step e) comprises comparing the at least one parameter of the other configuration to a predefined threshold, such as the at least one parameter of the predefined configuration.

**6.** Blood pressure monitoring system according to any of claims 3 - 5, further comprising:
- an output module, wherein the processor is operatively connected to the output module, and
wherein the processor is configured to:
f) control the output module to output the indicator for quality.

**7.** Blood pressure monitoring system according to the previous claim, wherein the output module is a visual and/or auditory output module.

**8.** Blood pressure monitoring system according any of the preceding claims, wherein the processor is configured to repeat steps a), b) on the one hand, and c) and/or d) on the other hand, throughout a continuous blood pressure monitoring procedure.

**9.** Blood pressure monitoring system according to any of the preceding claims, wherein the blood pressure monitoring system is portable and/or wherein the pressure measurement unit comprises a finger cuff.

**11.** Blood pressure monitoring system according to the previous claim, wherein the controllable element comprises is a bladder.

**12.** Blood pressure monitoring system according to the previous claim, wherein the controller is configured to control a volume of the bladder.

**13.** Blood pressure monitoring system according to any of the preceding claims, wherein the one or more sensors include at least one first sensor of a first type, and at least one second sensor of a second type, the second type being different than the first type.

**14.** Blood pressure monitoring system according to the previous claim, wherein the controller is configured to use a value from the at least one second sensor as an input, and wherein the controllable element is configured to influence, together with the body part, the value read by the at least one first sensor.

**15.** Blood pressure monitoring system according to claims 12 - 14, wherein the at least one first sensor is configured to measure a pressure inside the bladder and/or wherein the at least one second sensor is configured to measure an intensity of optionally visible light interacting with the body part.

**16.** Blood pressure monitoring system according to any of the preceding claims, wherein the data comprises a value representative of a physical quantity associated with the controllable element, such as a gas pressure inside the bladder of claim 11.

**17.** Blood pressure monitoring system according to any of the preceding claims, wherein the data comprises pressure data, such as blood pressure data.

**18.** Blood pressure monitoring system according to the previous claim, wherein the processor is further configured to control the output module to output the blood pressure simultaneous with the corresponding indicator for the quality.

**19.** Method of facilitating blood pressure measurements using a blood pressure measurement unit, the method comprising:
- controlling a controllable element of the blood pressure measurement unit according to a predefined configuration including at least one parameter; and
wherein the method further comprises:
a) receiving data from the blood pressure measurement unit,
b) determining another configuration for the controller based on the received data, by determining a value for the at least one parameter thereof; and
c) causing the controller to control the controllable element based on the other configuration instead of on the predetermined configuration.

**20.** Method of facilitating blood pressure measurements according to the previous claim, wherein the method further comprises:
d) determining a blood pressure of a user based on the data.

**21.** Method of facilitating blood pressure measurements according to any of the preceding method claims, the method further comprising:
e) determining an indicator of the quality of the data based on the other configuration.

**22.** Method of facilitating blood pressure measurements according to any of the preceding method claims, the method further comprising:
f) outputting the indicator of the quality.

**23.** Method of facilitating blood pressure measurements according to any of the preceding method claims, further comprising:
g) determining a suggested action for a user to improve the quality of the blood pressure measurements, and optionally outputting the suggestion to e.g. the user.

**24.** Method according to any of the preceding method claims, wherein the method is performed repeatedly during continuous blood pressure monitoring.
